# EUROPEAN PATENT APPLICATION

(11) **EP 2 133 033 A1**
(43) Date of publication of application: **16.12.2009**
(21) Application number: 08158003.7
(22) Date of filing: 11.06.2008
(51) Int. Cl.: A61B 17/66, A61B 17/80, A61B 17/86

(54) **Alveolar distractor**

(71) Applicant: Surgi-Tec NV, 8200 Brugge (BE)
(72) Inventor: The designation of the inventor has not yet been filed
(74) Representative: Brants, Johan P.E.

(57) **Abstract**

The present invention relates to a medical device (1) for performing alveolar distraction of a mobilised alveolar segment comprising a distal fixing element (3) disposed with one or more attachment points (6, 6') for the mobilised alveolar segment (81); a proximal fixing element (2) disposed with one or more attachment points (5, 5', 5") for remaining alveolar bone (82); an extending means (4) joining the distal (3) and proximal (2) fixing elements, which extending means (4) extends linearly along a straight axis (7), the y-axis, whereby a distal attachment region (52) and a proximal attachment region (51), each bound by the respectively employed attachment points (5, 5', 5" or 6, 6'), are mutually offset along an x-axis, perpendicular to the y-axis. It also relates to a method for performing an alveolar distraction.

## Description

### FIELD OF THE INVENTION

The present invention lies in the field of alveolar distraction osteogenesis. More specifically it relates to a new device therefor and a method of performing alveolar distraction osteogenesis.

### BACKGROUND TO THE INVENTION

An implant prosthesis is used to replace lost teeth in the mouth of a subject by a medical practitioner. The practitioner strives to maintain an optimum function and aesthetic appearance by positioning the implant abutting the gingiva, and securely mounted using the alveolar bone.

However, the loss of a tooth may result in a concomitant loss of alveolar bone. A deficiency in the alveolar bone may affect the position and secure fixing of the implant, and the contour of the gingiva. For a secure implant the volume of alveolar bone and the gingiva needs to be maintained or restored.

Reconstruction of the deficient alveolar bone may be achieved using alveolar distraction osteogenesis. This process involves cutting a segment of the alveolar bone at the site of implant, and, using a distraction device, elevating the mobilised segment in a slow, incremental manner to the desired position. Bone is regenerated in the chamber, known as the distraction zone or chamber, created by the elevation. As distraction proceeds, there is an increase in alveolar bone volume, and also an expansion of the adjacent soft tissue *i.e.* the periosteum and gingiva.

Within 3 to 4 months after the start of distraction, the base of the implant can be inserted into the mobile segment. The practitioner may apply other reconstructive features if necessary.

A problem in the art with alveolar distraction is obtaining a sufficient size of mobilised segment. The height of the mobilised segment should be sufficient to avoid its fragmentation or reabsorption during mobilisation. However, if it is too high, less bone will remain in the original part, and a fracture may arise during cutting or distraction owing to a weakened jaw.

A typical alveolar distraction device comprises a linear threaded rod, and two fixing elements - one for the mobilised segment and one for the original part and which are positioned in a linear alignment above and below the distraction zone. The pressure applied between the mobilized segment and one for the original part needs to be carefully regulated by the practitioner, since the original part is weakened in the region of the cut. Incremental movement necessitates daily visits by the patient to the practioner so that the movement of the mobilised segment is not excessive.

It is an aim of the present invention to provide an alveolar distraction device that overcomes the possibility of fracture and allows the patient to self-adjust the distraction zone.

### SUMMARY OF THE INVENTION

One embodiment of the invention is a medical device (1) for performing alveolar distraction of a mobilised alveolar segment comprising:
- a distal fixing element (3) disposed with one or more attachment points (6, 6') for the mobilised alveolar segment (81),
- a proximal fixing element (2) disposed with one or more attachment points (5, 5', 5") for remaining alveolar bone (82),
- an extending means (4) joining the distal (3) and proximal (2) fixing elements,
   which extending means (4) extends linearly along a straight axis (7), the y-axis, whereby a distal attachment region (52) and a proximal attachment region (51), each bound by the respectively employed attachment points (5, 5', 5" or 6, 6'), are mutually offset along an x-axis, perpendicular to the y-axis.

Another embodiment of the invention is a medical device as described herein, wherein the proximal fixing element (2) comprises a proximal bracket (10) for attachment to the stable alveolar segment and an arm (9) mechanically attached to the extending means (4), which arm (9) is mechanically joined to the proximal bracket (10).

Another embodiment of the invention is a medical device as described herein, wherein the distal fixing element (3) comprises a distal bracket (12) for attachment to the mobilised alveolar segment and an arm (11) mechanically attached to the extending means (4), which arm (11) is mechanically joined to the distal bracket (12).

Another embodiment of the invention is a medical device as described herein, wherein the extending means (6) is flanked, along the x-axis, by said brackets (10, 12) of the proximal (2) and distal (3) fixing elements.

Another embodiment of the invention is a medical device as described herein, wherein the proximal fixing element (2) comprises a microplate for attachment to the stable alveolar segment.

Another embodiment of the invention is a medical device as described herein, wherein the distal fixing element (3) is formed from a single element of dimensions to minimise distortion during implantation and is use.

Another embodiment of the invention is a medical device as described herein, whereby the proximal fixing element (2) or distal fixing element (3) is made at least partly of stainless steel, titanium, titanium alloy or a bioresorbable material.

Another embodiment of the invention is a medical device as described herein, whereby proximal bracket (10) is aligned to the left of the distal bracket (12) *in situ.*

Another embodiment of the invention is a medical device as described herein, whereby proximal bracket (10) is aligned to the right of the distal bracket (12) *in situ.*

Another embodiment of the invention is a medical device as described herein, whereby the extending means comprises a threaded rod, configured to move linearly the position of the distal fixing element (3), proximal fixing element (2) or both (2, 3) upon rotation.

Another embodiment of the invention is a medical device as described herein, whereby the extending means comprises a rack and pinion where the rack is attached to the distal fixing element (3), while the pinion is co-operatively coupled to the proximal fixing element (2) or *vice versa.*

Another embodiment of the invention is a medical device as described herein, whereby the extending means comprises a worm and pinion where the rack is attached to the distal fixing element 3, while the worm is co-operatively coupled to the proximal fixing element 2 or *vice versa.*

Another embodiment of the invention is a medical device as described herein, whereby the distal bracket (12) comprises one or more rotating, threaded keepers that can engage with the thread of an alveolar segment fixing screw, which threaded keeper can be freely rotated to translate the fixing screw in a direction along the z-axis without rotation of said screw.

Another embodiment of the invention is an extending means (4) comprising a coupling for a distal (3) fixing elements and a coupling for a proximal (2) fixing elements, which extending means (4) is configured to move linearly along straight axis, the y-axis, and is configured to adjust the distance between distal and proximal fixing element couplings.

Another embodiment of the invention is the extending means (4) as described herein comprising one or more features of the above embodiments.

Another embodiment of the invention a use of a medical device (1) as described herein for performing an alveolar distraction of a mobilised alveolar segment.

Another embodiment of the invention a method for performing an alveolar distraction osteogenesis comprising the steps of:
1) cutting a mobilized alveolar segment (81),
2) attaching a distal fixing element to the mobilized alveolar segment,
3) attaching a proximal fixing element to the remaining alveolar bone, in a stable region,
4) adjusting the vertical distance between the proximal and distal fixing elements using an extending means in mechanical connection with the proximal and distal fixing elements,
5) removing the fixing elements.

Another embodiment of the invention a method as described herein, wherein the method is performed using a device is defined elsewhere herein.

### FIGURE LEGENDS

**FIG. 1** Three-dimensional schematic view of an alveolar distraction device of the present invention.
**FIG. 2** Front schematic view of the alveolar distraction device as shown in FIG. 1, where the extending means **4** is retracted.
**FIG. 3** Front schematic view of the alveolar distraction device as shown in FIG. 1, where the extending means **4,** is partially extended.
**FIG. 4** Front schematic view of the alveolar distraction device as shown in FIG. 1, where the attachment regions are shaded.
**FIG. 5** Top schematic view of the alveolar distraction device as shown in FIG. 4, where the same attachment regions are shaded.
**FIG. 6 to 10****:** Schematic views of a lower jaw with a deficient alveolar ridge which is corrected using a device and method of the present invention. FIG. 7 shows the horizontal and vertical components of a cut, FIG. 8 shows a mobilized alveolar segment, FIG. 9 shows a device of the present invention attached, FIG. 10 shows a device of the present invention effecting a distraction of the mobilised alveolar segment.
**FIG. 11** shows a three-dimensional schematic view of an alveolar distraction device of the present invention showing the rotating, threaded keepers present in one of the brackets.

### DETAILED DESCRIPTION OF THE INVENTION

Unless defined otherwise, all technical and scientific terms used herein have the same meaning as is commonly understood by one of skill in the art. All publications referenced herein are incorporated by reference thereto. All United States patents and patent applications referenced herein are incorporated by reference herein in their entirety including the drawings.

The articles "a" and "an" are used herein to refer to one or to more than one, *i.e.* to at least one of the grammatical object of the article. By way of example, "a hole" means one hole or more than one hole.

The recitation of numerical ranges by endpoints includes all integer numbers and, where appropriate, fractions subsumed within that range (*e*.*g*. 1 to 5 can include 1, 2, 3, 4 when referring to, for example, a number of samples, and can also include 1.5, 2, 2.75 and 3.80, when referring to, for example, measurements). The recitation of end points also includes the end point values themselves (*e*.*g*. from 1.0 to 5.0 includes both 1.0 and 5.0)

Reference is made in the description below to the drawings which exemplify particular embodiments of the invention; they are not at all intended to be limiting. The skilled person may adapt the device and method described herein, and substituent or add components, features and steps according to the common practices of the person skilled in the art.

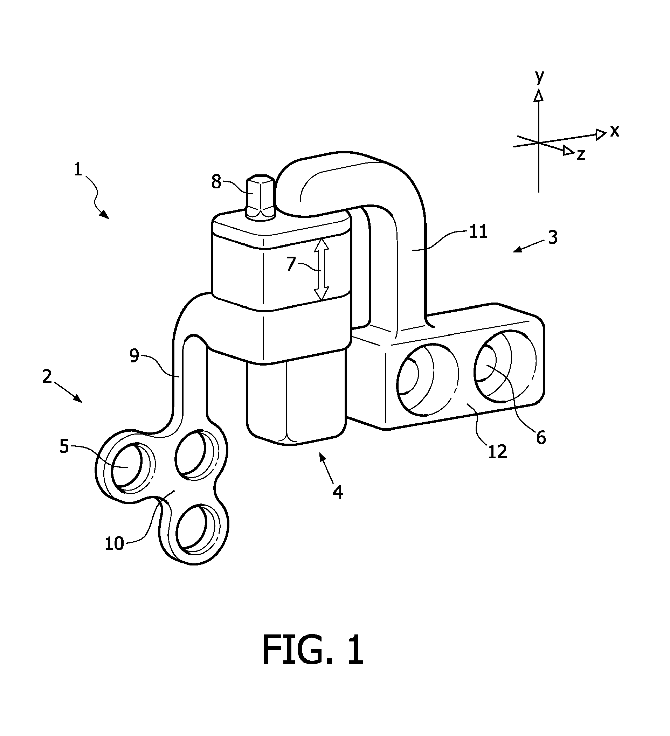

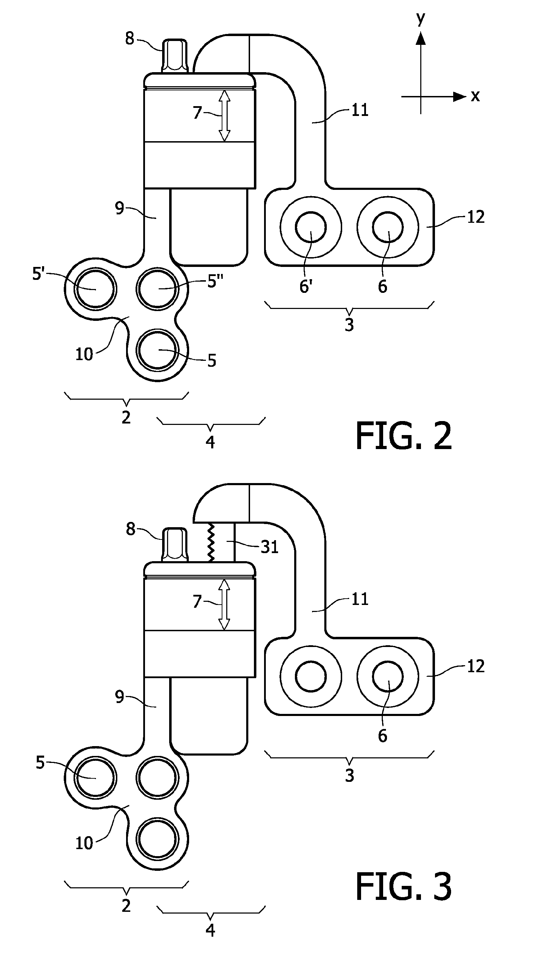

The alveolar distraction device **1** (**FIGS. 1** and **2**) of the present invention modifies the separation between the mobilised segment and remaining alveolar bone during alveolar distraction osteogenesis. The device is capable of being placed intra-orally and is attached to the mobilised alveolar segment and the remaining alveolar bone in the subject.

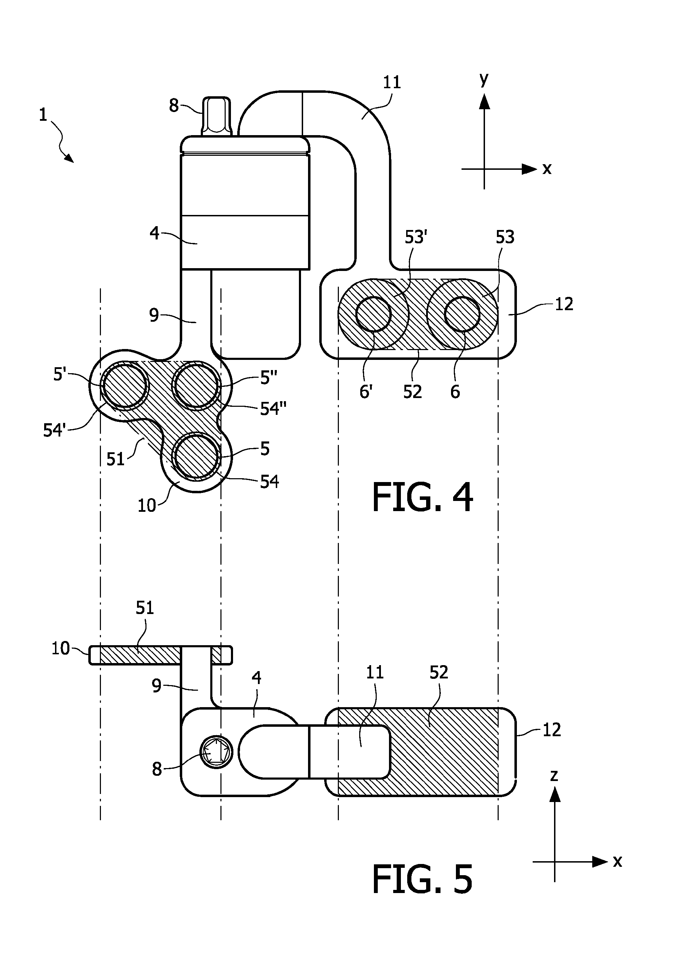

The device **1** comprises two alveolar fixing elements, one (known as the distal fixing element **3**) for attachment to the mobilised alveolar segment and one (known as the proximal fixing element **2**) for attachment to the remaining alveolar bone in the subject, each fixing element comprising with a bracket **12, 10** adapted to connect the fixing element to the respective alveolar segment. The bracket **12, 10** comprises an attachment region **52, 51** (**FIGS. 4** and **5**), that is a region bound by the employed attachment points.

The alveolar distraction device **1** of the present invention further comprises an extending means **4** joining the distal **3** and proximal **2** fixing elements, which extending means **4** moves linearly along a straight axis **7,** (known as the y-axis) and is configured to adjust the distance between distal and proximal fixing elements. The modification of the distance along the y-axis between the fixing elements may be made once the device **1** is implanted in the patient; the device may also permit modification of the orientation of the footplates in relation to each other before implantation. The entire device is located intra-orally in the patient.

According to the present invention, the attachment region **52** of the distal fixing element **3** is displaced or offset from the attachment region **51** of the proximal fixing element along the axis perpendicular to the axis of extension (y-axis). In other words, the distal attachment region **52** and a proximal attachment region **51,** bound by the respectively employed attachment points **5, 5', 5"** or **6, 6',** are mutually offset along the x-axis, perpendicular to the y-axis. In front view (*i.e.* along the z-axis) or plan view (*i.e.* along the y-axis) the attachment region **52** of the distal fixing element and the attachment region **51** of the proximal fixing element do not overlap (**FIG. 5**). In plan view the attachment region **52** of the distal fixing element **12** is displaced or offset from the attachment region **51** of the proximal fixing element **10** along an x-axis, which axis is perpendicular to the y-axis.

The attachment region **52** of the distal fixing element **3** is displaced along the x-axis from the attachment region **51** of the proximal fixing element **2** to a degree that allows attachment of the proximal fixing element to a stabilised region of the alveolar, known herein as the stable alveolar segment. The displacement is contrary to the prior art where the distal and proximal fixing elements are aligned along the y-axis, so applying compression forces directly to the weakened portion (known herein as the weakened alveolar segment) of the alveolar. By using a displaced arrangement, considerably more force can be applied, allowing larger a mobilised segment to be cut by the practitioner and greater daily incremental movements.

The attachment region **52** of the distal fixing element **3** is typically displaced along the y-axis from the attachment region **51** of the proximal fixing element **2** when the extending means **4** is in a closed position. The separation along the y-axis between the respective attachment regions **51, 52** will increase as the extending means extends (**FIG. 2** cf **FIG. 3**).

The stable alveolar segment is a region where the height of remaining bone is not less than 15 mm, the height being measured from the base of the jaw to the base of the horizontal. It is typically greater than 6, 7, 8, 9, 10, 11, 12, 13, 14, 15, 16, 17, 18, 19, 20 mm distant from the border of the cut.

### Proximal fixing element

Proximal fixing element **2** comprises a bracket **10** for attachment to the stable alveolar segment and an arm **9** mechanically attached to the extending means **4,** which arm **9** is mechanically joined to the bracket **10** (known as the proximal bracket herein); said mechanical joins and attachments are preferably rigid. The bracket **10** of the proximal fixing element **2** is disposed with one or more (*e*.*g*. 1, 2, 3, 4 or 5 or more) screw holes **5** to receive screws for attachment to the stable alveolar segment. The central axes of the screw holes run essentially along the z-axis. The fixing element is preferably made from a single element.

The proximal bracket **10** comprises an attachment region **51,** that is a region bound by the employed attachment points. This is illustrated in **FIG. 4** which depicts the boundary of the attachment region **51** defined by the employed screw holes **5, 5', 5".** The skilled person will understand that the boundary may be extended, when the employed screws have a wide head, to include the region of the head. This is indicated in **FIG.4** where the respective attachment regions are extended to include a countersunk (**53, 53', 54, 54', 54"**) portion which engages with the screw head.

The arm **9** of the proximal fixing element **2** is shaped to align the bracket **10** with the stable alveolar segment. It may comprise one or more bends (see **FIG. 1**) to achieve this alignment. Since the bracket **10** of the proximal fixing element **2** is displaced with respect to that of the distal fixing element **2** as described above, the arm **9** may be more or less extended along the x-axis compared with the other arm **11.** **FIG. 2** clearly show the instance when the arm **9** of the proximal fixing element **2** is not extended along the x-axis direction, compared with arm **9** of the distal fixing element **3.** The skilled person will understand the configuration could equally be reversed *i.e.* the arm of the proximal fixing element **2** may be more extended along the x-axis direction compared with arm of the distal fixing element **3,** to achieve the same juxtaposition. The relative length of the arms will depend on the placement of the extension means *e*.*g*. centrally disposed, or to the left or to the right of the fixing elements **2, 3.**

For example, displacement may be achieved by attaching the respective fixing elements **2, 3** to the extending means **4** such that their arms **9, 11** extend from opposing sides of the extending **4** means as shown in the **FIG. 2****.** This configuration places the extending means **6** in a central part, flanked by the brackets of the proximal **2** and distal **3** fixing elements. In this arrangement, the spacing is principally achieved by the centrally disposed extending means **6.**

The proximal fixing element **10** is generally below the level of the distal fixing element **12.** It is an aspect of the invention that the proximal attachment region **51** is below the distal attachment region **52** when viewed front the front of the device (**FIG. 4**). It is an aspect of the invention that the proximal attachment region **51** is below the distal attachment region **52** when viewed front the front of the device (**FIG. 4**), and the regions **51, 52** do not overlap along the y-axis.

The proximal fixing element **2** is mechanically attached to the extending means **4** *via* the arm **9.** The attachment between the arm **9** and extending means **4** may be permanent (*e*.*g*. welding, molded) or reversible (*e*.*g*. using a screw fitting, push fitting or other type of suitable coupling). Preferably, the proximal fixing element **2** is mechanically attached to the extending means **4** rigidly, such that it exhibits no movement relative to the extending means **4** other than a relative translation **7** along the y-axis. It is an aspect of the invention that the proximal fixing element **2** cannot be articulated around an axis parallel to the y-axis. However, proximal fixing element **2** may be made partly from a material that can be shaped by the practitioner to adapt to the curvature of the jaw, for example.

The proximal fixing element **2** is typically designed for subcutaneous implantation and attachment to the stable alveolar segment. The proximal fixing element **2** may be made at least partly of any suitable material such as stainless steel, titanium, or titanium alloy. Alternatively, it may be composed at least partly of a bioresorbable material. The proximal fixing element **2** may be formed from a microplate, known in the art, which is a thin, continuous strip of rigid, but malleable material, disposed with a plurality screw holes in the bracket region **10,** and can be manipulated to form a desired shape, which shape is retained *in situ.* The bracket of the microplate is generally more malleable than the arm. Typically, the practitioner can bend the microplate using the force a pair of pliers. The microplate is sufficiently thin to allow implantation underneath the gum, typically between 0.2 to 0.9 mm thick, preferably 0.7 mm thick.

### Distal fixing element

Distal fixing element **3** comprises a bracket **12** for attachment to the mobilised alveolar segment and an arm **11** mechanically attached to the extending means **4,** which arm is mechanically joined to the bracket **12**; said mechanical joins and attachments are preferably rigid. The bracket **12** of the distal fixing element **3** is disposed with one or more (*e*.*g*. 1, 2, 3, 4 or 5) screw holes **6** to receive screws for attachment to the mobilised alveolar segment. The central axes of the screw holes run essentially along the z-axis. The fixing element is preferably made from a single element.

The arm **11** of the distal fixing element **3** is shaped to align the bracket **12** with the mobilised alveolar segment. It may comprise one or more bends to achieve the alignment. Since the bracket **12** of the distal fixing element **3** is displaced with respect to that of the proximal fixing element **2** as described above, the arm **11** may be more or less extended along the x-axis compared with the other arm. **FIG. 2** shows the instance when the arm **11** of the distal fixing element **3** is more extended along the x-axis direction compared with arm **9** of the proximal fixing element **2.** The skilled person will understand the configuration could equally be reversed *i.e.* the arm **11** of the distal fixing element **3** may be less extended along the x-axis direction compared with arm **9** of the proximal fixing element **2,** to achieve the same juxtaposition. As explained above, the relative length of the arms will depend on the placement of the extension means **4** *e*.*g*. centrally disposed, or to the left or to the right of the fixing elements **2, 3.**

The distal bracket **12** comprises an attachment region **52,** that is a region bound by the employed attachment points. This is illustrated in **FIG. 4** which depicts the boundary of the attachment region **52** defined by the employed screw holes **6, 6'.**

For example, the displacement may be achieved by attaching the respective fixing elements **2, 3** to the extending means **4** such that their arms extend from opposing sides of the extending means. This configuration places the extending means **6** in a central part, flanked by the brackets of the proximal **2** and distal **3** fixing elements. In this arrangement, the displacement is principally achieved by the centrally disposed extending means **6.**

The distal fixing element **12** is generally above the level of the proximal fixing element **10** when viewed front the front of the device (**FIG. 4**). It is an aspect of the invention that the distal attachment region **52** is above the proximal attachment region **51** when viewed front the front of the device (**FIG. 4**). It is an aspect of the invention that the distal attachment region **52** is above the proximal attachment region **51** when viewed front the front of the device (**FIG. 4**), and, the regions **51, 52** do not overlap along the y-axis.

The bracket **12** of the distal fixing element **3** is configured to attach to the mobilised alveolar segment. Beside the extending means **4** providing movement of the mobilised alveolar segment along the y-axis, the bracket **12** may permit translational movement of the alveolar segment along the z-axis. This enables the mobilised alveolar segment to be moved and optionally locked in a particular dorsal (back) or ventral (front) direction.

Translation along the z-axis can be achieved by the use of fixing screws, for attachment to the mobilised alveolar segment, the shafts of which are configured to slot into non-threaded holes **6, 6'** present in the bracket **12.** Since the holes **6, 6'** are not threaded, the shaft of the fixing screws can move freely in the dorsal or ventral directions within the holes **6, 6'.** Where headed fixing screws are used, the length of the shaft is sufficiently long to permit dorsal or ventral movement without restriction by the screw head contacting the front of the bracket **12.** The final position of the mobilised alveolar segment along the z-axis can be fixed by using a locking screw present in the bracket, which tip enters the screw hole **6, 6'** and abuts the shaft of the fixing screw.

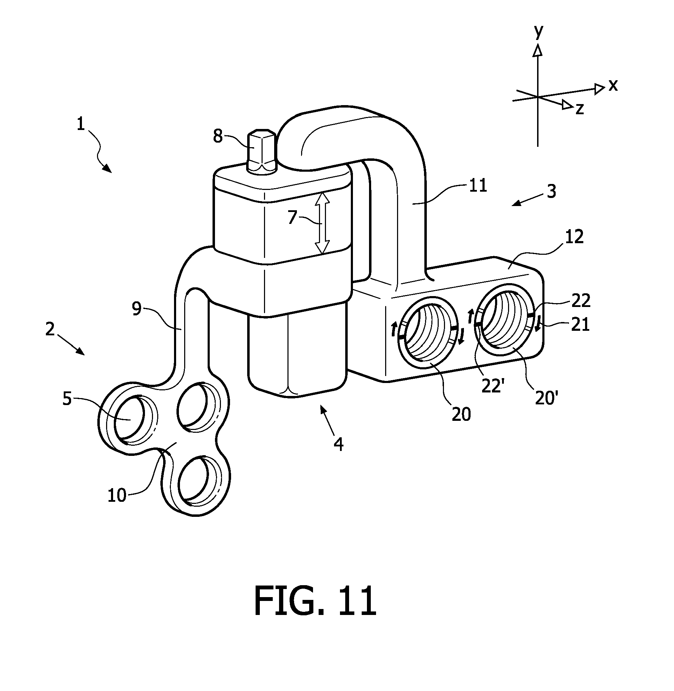

In an alternative embodiment, translation along the z-axis can be achieved by the use of fixing screws, for attachment to the mobilised alveolar segment, the shafts of which are configured to slot into rotating, threaded keepers **20, 20'** present in the bracket **12** (**FIG. 11**). A rotating threaded keeper **20, 20'** is essentially a threaded element (*e*.*g*. threaded nut or ring) that can engage with the thread of a fixing screw, but which keeper is attached to the bracket **12** and can freely rotate. Rotation **21** of the threaded keeper allows translation of the fixing screw in a direction along the z-axis without rotation of said screw. The keeper **20, 20'** may be provided with one or more tabs **22, 22',** that provides a gripping point allowing the practitioner to manually rotate or hold the keeper **20, 20'.** The tab **22, 22'** can be a ridge or a groove, for example, in the ventral surface of the ring keeper **20, 20'.** Thus, the tab **22, 22'** can be held to prevent the keeper **20, 20'** from rotating while the fixing screws are inserted through the bracket and driven into the mobilised alveolar segment. Once in place, each keeper **20, 20'** is rotated using the tab **22, 22',** which movement translates the mobilised alveolar segment in a direction along the z-axis. This system takes advantage of the threaded fixing screw, providing an exquisite bi-directional adjustment with a minimum of protruding components.

The distal fixing element **3** is mechanically attached to the extending means **4** *via* the arm **11.** The attachment may be permanent (*e*.*g*. welding, molded) or reversible (*e*.*g*. screw fitting, push fitting or other type of suitable coupling). Preferably, the distal fixing element **3** is mechanically attached to the extending means rigidly, such that it exhibits no movement relative to the extending means other than a relative translation **7** along the y-axis. It is an aspect of the invention that the distal fixing element **3** cannot be articulated around an axis parallel to the y-axis. However, it may be made partly from a material that can be shaped by the practitioner to adapt to a curvature in the mobilised alveolar segment, for example.

The distal fixing element **3** is designed for attachment to the mobilised alveolar segment. The distal fixing element **3** may be made at least partly of stainless steel, titanium, or titanium alloy. Alternatively, it may be composed at least partly of a bioresorbable material. According to one aspect of the invention, the distal fixing element **3** is formed from a rigid material; its shape cannot be manipulated by the practitioner. The arm and bracket of the distal fixing element are preferably formed from a single element, and of thickness to minimise distortion during implantation and is use.

Alternatively, and less preferred, the distal fixing element **3** may be formed from a micro plate as described above, which is a thin strip of rigid malleable material, disposed with a plurality screw holes, and can be manipulated into a desired shape using the force a pair of pliers. The micro plate may sufficiently thin to allow implantation underneath the gum tissue, typically between 0.2 to 0.9 mm thick, preferably 0.7 mm thick.

### Extending means

The extending means **4** is mechanically joined to the distal **3** and proximal **2** fixing elements, which extending means **4** moves linearly along a straight axis, (known as the y-axis) and is configured to adjust the distance between distal and proximal fixing elements. **FIG. 2** shows the distal **3** and proximal **2** fixing elements in at a minimum distance apart; **FIG.3** shows the distal **3** and proximal **2** fixing elements separated along the y-axis *via* a rack **31,** in this instance, of the extending means **4.** The position of the attachment, combined with the shape of the arms of the proximal **2** and distal **3** fixing elements allow the respective brackets to align *in situ* with the stable and mobile alveolar segments respectively.

As already mentioned above, the attachment region **52** of the distal fixing element is displaced from the attachment region **51** of the proximal fixing element along the axis perpendicular to the axis of extension. In plan view (*i.e.* along the y-axis) or in front view (*i.e.* along the z-axis) the attachment region **52** of the distal fixing element and the attachment region **51** of the proximal fixing element do not overlap (**FIG. 5**). In plan view or in front view the attachment region **52** of the distal fixing element is displaced from the attachment region **51** of the proximal fixing element along an x-axis, which is perpendicular to the y-axis (**FIG. 5**).

According to one aspect of the invention, the minimum distance between the proximal **10** and distal **12** brackets as measured along the x-axis is 10mm, 12mm, 14mm, 16mm, 18mm, 20mm, or a value in the range between any two of the aforementioned values.

Depending on where the fixing elements are attached the extending means **4,** the proximal bracket **10** may be aligned to the left of the distal bracket **12,** *in situ* and/or as observed from the front face of the device **1.** Alternatively, the proximal bracket **10** may be aligned to the right of the distal bracket **12,** as observed *in situ* and/or from the front face. A left- or right-positioned proximal bracket **10** is achieved depending on where the respective fitting elements **2, 3** are attached to the extending means. The choice of a left- or right-positioned proximal bracket **10** may depend on the intra-oral space available to the practitioner. **FIG. 2** shows a proximal bracket **10** positioned to the left of the distal bracket **12.** The front face of the device **1** is shown in **FIG. 2****,** which face does not contact the gum *in situ.* The front face is normally viewed in the upright orientation of the device, which orientation places y-axis along the direction of distraction and the distal bracket **12** at least partly above the proximal bracket **10.**

The extending mechanism of the extending means **4** can be any known in the art. It should enable a precise control of the distance between the distal **3** and proximal **2** fixing elements, which distance can be locked between incremental movements.

The most widely known extending means makes use of threaded rod, which by turning, linearly moves the position of the distal fixing element **3,** proximal fixing element **2** or both **2, 3.** According to one embodiment of the invention, the extending means **4** comprises a lead screw that has external threading. The distal fixing element **3,** the proximal fixing element **2** or both **2, 3,** comprise a bore with an internal thread that engages the external thread of the lead screw. Rotation of the lead screw is translated into linear movement, so that the distance between the distal fixing element **3** and the proximal fixing element **2** is adjusted. In the case where both the distal fixing element **3** and the proximal fixing element **2** engage with the lead screw, the respective bores may be threaded in opposite directions; rotation of the lead screw is translated into linear movement along the lead screw, of both the distal fixing element **3** and the proximal fixing element **2** but in opposite directions. In the case where either the distal fixing element **3** or the proximal fixing element engages **2** with the lead screw, but not both, the non-engaging fixing element is attached to the lead screw using a conventional rotary coupling.

According to another embodiment of the invention, the extending means **4** comprises a rack-and-pinion assembly. The rack element may carry the distal fixing element **3,** while the pinion may carry the proximal fixing element **2** or *vice versa.* The rack may be attached to the distal fixing element **3,** while the pinion may be co-operatively coupled the proximal fixing element **2** or *vice versa.* The rack element is normally oriented parallel to the y-axis. The invention also includes variants where rack element is set at an angle to the y-axis. The turning axis of the pinion may be extended to form an accessible adjuster. Rotation of the adjuster, turns the pinion. Rotation of the pinion, for example by using a key brings about the required change in distance between the distal fixing element **3** and the proximal fixing element **2** by linear movement of the rack. Other elements can be present within the extending means, which allow fine control of pinion rotation *e*.*g*. a rachet system, or a locking mechanism. Using the rack and pinion assembly, the axis of rotation of the pinion is perpendicular to the axis of translation of the rack (y-axis); therefore, access to the adjuster will be from the front of the device

According to another embodiment of the invention, the extending means **4** comprises a worm-and-rack assembly. The rack element may carry the distal fixing element **3,** while the worm part may carry the proximal fixing element **2** or *vice versa.* The rack may be attached to the distal fixing element **3,** while the worm may be co-operatively coupled the proximal fixing element **2** or *vice versa* The rack element is normally oriented parallel to the y-axis. The invention also includes variants where rack element is set at an angle to the y-axis. The turning axis of the worm may be extended to form an accessible adjuster **8.** Rotation of the adjuster **8** turns the worm. Rotation of the worm, for example by using a key brings about the required change in distance between the distal fixing element **3** and the proximal fixing element **2** by linear movement of the rack. Other elements can be present within the extending means, which allow fine control of worm rotation *e*.*g*. a rachet system, or a locking mechanism. Using the worm and rack assembly, the axis of rotation of the pinion is parallel to the axis of translation of the rack (y-axis); therefore, access to the adjuster **8** will be from the top of the device; this arrangement is shown in the FIGS. Rotation of the adjuster **8** in **FIG. 2** leads to a linear movement of the rack **31** (**FIG. 3**), and separation of the respective fixing elements **2, 3.**

The extending means **4** may be made at least partly of stainless steel, titanium, or titanium alloy.

One embodiment of the invention is an extending means **4** comprising a coupling for a distal **3** fixing elements and a coupling for a proximal **2** fixing elements, which extending means **4** is configured to move linearly along a straight axis, (known as the y-axis) and is configured to adjust the distance between distal and proximal fixing element couplings. The extending means **4** may be devoid of the proximal **2** and/or distal **3** fixing elements. The extending means **4** so configured may be re-usable. The details and configurations of the extending means given above apply to the re-usable extending means **4.**

### Dummy arm

In some cases, a portion of mobilized alveolar segment may be elongated in an x or y direction or both, giving rise to a large fragment. In some circumstances it may be necessary to passively support the mobilized alveolar segment using a dummy arm. A dummy arm is a hinged or flexible arm with a bracket for attachment to the mobilized alveolar segment and a bracket for attachment to the stable alveolar segment. It provides an additional support to the mobilized alveolar segment by connecting it to the stable alveolar segment in a region most distant from the point of attachment of the present device. The dummy arm is passive, meaning it has no adjustment means, but owing to its hinged or flexible character, it adapts as the mobilized alveolar segment is moved.

The device **1** of the present invention advantageously provides a means to perform alveolar distraction osteogenesis which minimized risk of fracture to the remaining alveolar bone. It also permits a larger mobilized alveolar segment which has more areas available for attachment; the larger mobilized alveolar segment is also unlikely to fracture during fitting and distraction. Because it is no longer necessary to carefully monitor the tensions applied by the device, incremental movements can safely be performed by the patient at home. This avoids repeated, daily visits to the practitioner, saving time for all parties and reducing costs of treatment. The device also reduces infections and allows a larger movement of the alveolar segment.

### Method

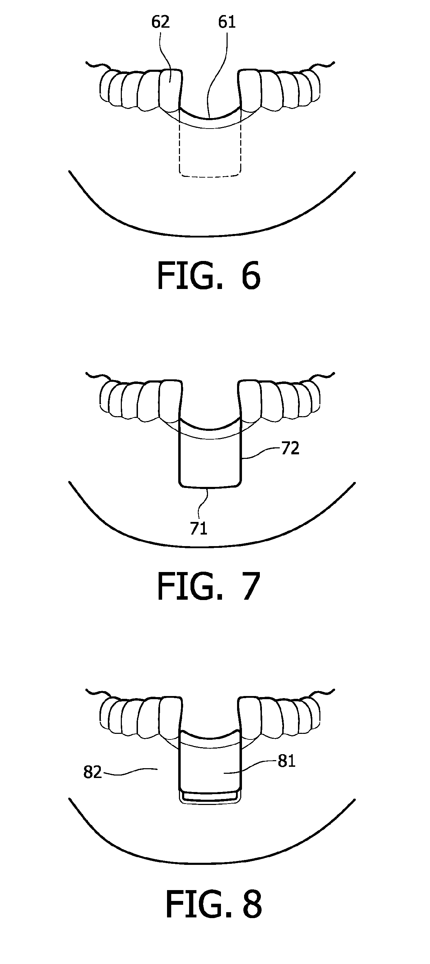

The present invention also includes a method for alveolar distraction osteogenesis using the above described device for distraction of the mobilised alveolar fragment. Reference is made herein to **FIGS. 6** to **10****.**

**FIG. 6** shows a view of a lower jaw disposed with natural teeth **62.** A deficient alveolar ridge **61** is apparent after loss of the lower front teeth. According to the method, a mobilised alveolar fragment is cut from the lower jaw, which section is moved vertically upwards in periodic increments.

A saw (*e*.*g*. microsaggital saw and/or micro-oscillating saw) is used to cut the mobilised alveolar fragment. Generally the mobilised alveolar fragment is cut using two vertical cuts joined by one horizonal cut. The space between the vertical cuts **71** (**FIG. 7**) is determined by the width of the teeth gap. The length **72** of the vertical cuts determines the height of the mobilised alveolar fragment, and the amount of remaining alveolar bone. Because the present invention attaches to a stable alveolar segment, the length of the vertical cuts can be increased, without a risk of fracturing the weakened alveolar segment.

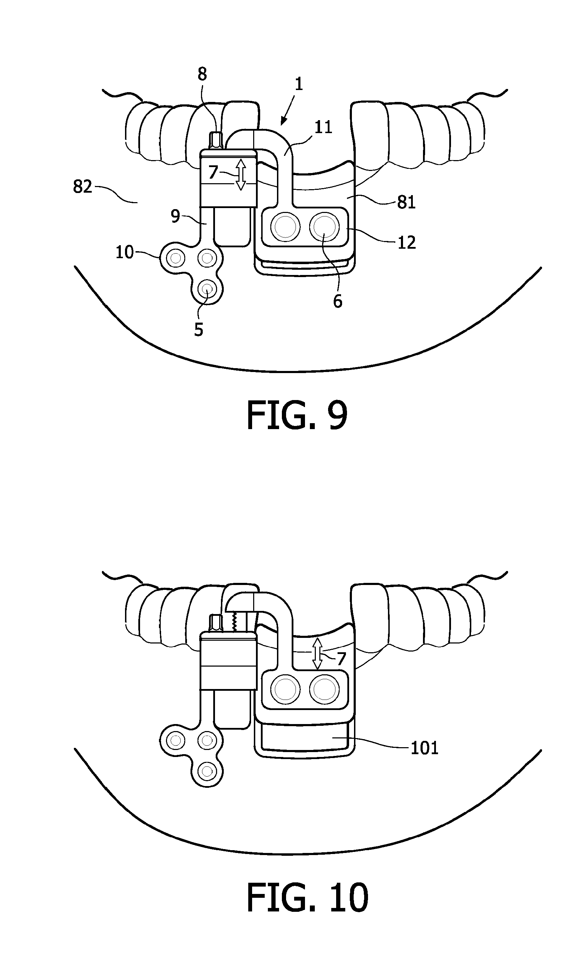

After the mobilised alveolar segment **81** (**FIG. 8**) has been cut, it can be freed from the remaining alveolar bone **72** using a suitable tool (*e*.*g*. an osteotome). The device of the present invention is attached to the mobilised alveolar segment **91** (**FIG. 9**) and to the stable alveolar segment **82.** Because of the displacement of the respective brackets along the x-axis, the present device avoids attachment to the weakened alveolar segment **91.** The device of the present invention is aligned so that the axis of extension (y-axis) is aligned with the direction of distraction. Turning the adjuster on the device changes the distance between the distal and proximal fixing elements, so adjusting the volume of distraction chamber **101** (**FIG. 10**).

One embodiment of the present invention is a use of the present device for alveolar distraction osteogenesis.

Another embodiment of the present invention is a method for performing an alveolar distraction osteogenesis comprising the steps of:
1) cutting a mobilized alveolar segment **81,**
2) attaching a distal fixing element to the mobilized alveolar segment,
3) attaching a proximal fixing element to the remaining alveolar bone, in a stable region,
4) adjusting the vertical distance between the proximal and distal fixing elements using an extending means in mechanical connection with the proximal and distal fixing elements,
5) removing the fixing elements.

Another embodiment of the present invention is a method as described above using a device of the present invention.

## Claims

1. A medical device (1) for performing alveolar distraction of a mobilised alveolar segment comprising:
- a distal fixing element (3) disposed with one or more attachment points (6, 6') for the mobilised alveolar segment (81),
- a proximal fixing element (2) disposed with one or more attachment points (5, 5', 5") for remaining alveolar bone (82),
- an extending means (4) joining the distal (3) and proximal (2) fixing elements,
which extending means (4) extends linearly along a straight axis (7), the y-axis, whereby a distal attachment region (52) and a proximal attachment region (51), each bound by the respectively employed attachment points (5, 5', 5" or 6, 6'), are mutually offset along an x-axis, perpendicular to the y-axis.

2. Device according to claim 1, wherein the proximal fixing element (2) comprises a proximal bracket (10) for attachment to the stable alveolar segment and an arm (9) mechanically attached to the extending means (4), which arm (9) is mechanically joined to the proximal bracket (10).

3. Device according to claim 1 or 2, wherein the distal fixing element (3) comprises a distal bracket (12) for attachment to the mobilised alveolar segment and an arm (11) mechanically attached to the extending means (4), which arm (11) is mechanically joined to the distal bracket (12).

4. Device according to any of claims 1 to 3, wherein the extending means (6) is flanked, along the x-axis, by said brackets (10, 12) of the proximal (2) and distal (3) fixing elements.

5. Device according to any of claims 1 to 4, wherein the proximal fixing element (2) comprises a microplate for attachment to the stable alveolar segment.

6. Device according to any of claims 1 to 5, wherein the distal fixing element (3) is formed from a single element of dimensions to minimise distortion during implantation and is use.

7. Device according to any of claims 1 to 6, whereby the proximal fixing element (2) or distal fixing element (3) is made at least partly of stainless steel, titanium, titanium alloy or a bioresorbable material.

8. Device according to any of claims 1 to 7, whereby proximal bracket (10) is aligned to the left of the distal bracket (12) *in situ.*

9. Device according to any of claims 1 to 7, whereby proximal bracket (10) is aligned to the right of the distal bracket (12) *in situ.*

10. Device according to any of claims 1 to 9, whereby the extending means comprises a threaded rod, configured to move linearly the position of the distal fixing element (3), proximal fixing element (2) or both (2, 3) upon rotation.

11. Device according to any of claims 1 to 9, whereby the extending means comprises a rack and pinion where the rack is attached to the distal fixing element (3), while the pinion is co-operatively coupled to the proximal fixing element (2) or *vice versa.*

12. Device according to any of claims 1 to 9, whereby the extending means comprises a worm and pinion where the rack is attached to the distal fixing element 3, while the worm is co-operatively coupled to the proximal fixing element 2 or *vice versa.*

13. Device according to any of claims 1 to 12 whereby the distal bracket (12) comprises one or more rotating, threaded keepers that can engage with the thread of an alveolar segment fixing screw, which threaded keeper can be freely rotated to translate the fixing screw in a direction along the z-axis without rotation of said screw.

14. An extending means (4) comprising a coupling for a distal (3) fixing elements and a coupling for a proximal (2) fixing elements, which extending means (4) is configured to move linearly along straight axis, the y-axis, and is configured to adjust the distance between distal and proximal fixing element couplings.

15. The extending means according to claim 14, comprising one or more features of claims 1 to 13.
